# EUROPÄISCHE PATENTSCHRIFT

(11) **EP 2 455 774 B1**
(45) Veröffentlichungstag und Bekanntmachung des Hinweises auf die Patenterteilung: **21.08.2013**
(21) Anmeldenummer: 10191838.1
(22) Anmeldetag: 19.11.2010
(51) Int. Cl.: G01R 33/12, G01N 15/06, G01N 33/28

(54) **Sensorvorrichtung und Verfahren zu deren Betrieb**
Sensor device and method for its operation
Dispositif de capteur et son procédé de fonctionnement

(43) Veröffentlichungstag der Anmeldung: 23.05.2012
(73) Patentinhaber: ARGO-HYTOS GmbH, 76703 Kraichtal (DE)
(72) Erfinder: Dyck, Harry, 76133, Karlsruhe (DE); Knebel, Matthias, 76684, Östringen (DE); Klosek, Heinrich, 76703, Kraichtal (DE); Meindorf, Thomas, 76789, Karlsdorf-Neuthard (DE)
(74) Vertreter: Hoeger, Stellrecht & Partner Patentanwälte

(56) Entgegenhaltungen:
- DE-A1- 10 339 905
- GB-A- 2 029 580
- US-A- 5 674 401

## Beschreibung

Die vorliegende Erfindung betrifft eine Sensorvorrichtung zur Detektion von ferromagnetischen Partikeln in einem Fluid.

Die Erfindung betrifft ferner ein Verfahren zum Betrieb einer derartigen Sensorvorrichtung.

Fluide wie Hydraulikflüssigkeiten und Schmiermittel, die in entsprechenden fluidtechnischen Anlagen eingesetzt werden, weisen fast immer eine mehr oder weniger starke Verschmutzung durch ferromagnetische Partikel, d.h. Eisen- bzw. Stahlpartikel, auf. Solche Partikel gelangen meist schon bei der Herstellung und Wartung der Anlage in das System, vor allem aber entstehen sie kontinuierlich durch Abrieb an beweglichen Komponenten während des Betriebs der Anlage (in besonders hohem Ausmaß z.B. bei Getrieben). Da eine zu hohe Partikelbelastung des Fluids die Funktionsfähigkeit der Anlage beeinträchtigen oder sogar zu einem Totalausfall führen kann, ist eine Überwachung der Partikelbelastung erforderlich, um bei Erreichen eines bestimmten Grenzwertes, der je nach Art der fluidtechnischen Anlage unterschiedlich sein kann, geeignete Maßnahmen ergreifen zu können (z.B. Austausch oder Reinigung von Partikelfiltern, Austausch des Fluids, Reinigung der gesamten Anlage usw.).

Es sind Sensoren zur Detektion von ferromagnetischen Partikeln bekannt, bei denen ein Permanentmagnet in das vorbeiströmende Fluid hineinragt, wobei sich in dem Fluid enthaltene ferromagentische Partikel an den Magneten anlagern. Diese Anlagerung führt zu einer Änderung des Magnetfeldes, welche mit einem geeigneten Detektor (z.B. einem Hall-Detektor) erfasst werden kann. Eine solche Vorrichtung wird z.B. in der GB 2 029 580 A beschrieben. Nachteilig bei diesen Sensoren ist die Tatsache, dass die an dem Permanentmagneten anhaftenden Partikel wieder entfernt werden müssen (spätestens wenn die maximale Kapazität des Magneten erreicht ist). Zu diesem Zweck muss der Sensor entweder ausgetauscht oder entfernt und manuell gereinigt werden, was einen relativ hohen Aufwand darstellt, oder die angelagerten Partikel werden mittels einer Bürste oder dergleichen entfernt, die als Teil des Sensors installiert ist. Ein Sensor mit solchen beweglichen mechanischen Komponenten ist aber aufwändig in der Herstellung und anfälliger für Fehlfunktionen.

Der Erfindung liegt daher die Aufgabe zugrunde, eine Sensorvorrichtung vorzuschlagen, die auf einfache Weise kontinuierlich betrieben werden kann.

Diese Aufgabe wird erfindungsgemäß gelöst durch eine Sensorvorrichtung der eingangs genannten Art, umfassend
- eine Sensorfläche, die dem Fluid ausgesetzt ist;
- einen Permanentmagnet, der ein Magnetfeld im Bereich der Sensorfläche erzeugt;
- einen Detektor, mit dem ferromagnetische Partikel im Bereich der Sensorfläche detektierbar sind;
- eine Induktionsspule, mit der ein Magnetfeld induzierbar ist, das dem Magnetfeld des Permanentmagneten entgegen gerichtet ist; und
- mindestens einen Kondensator, der über die Induktionsspule entladbar ist, wobei durch die Entladung des mindestens einen Kondensators über die Induktionsspule ein maximales Magnetfeld induzierbar ist, welches stärker ist als das Magnetfeld des Permanentmagneten.

Die Kernidee der Erfindung besteht darin, die anhaftenden Partikel nicht manuell bzw. mechanisch von dem Permanentmagneten zu entfernen, sondern dadurch, dass das Magnetfeld des Permanentmagneten durch ein induziertes, entgegen gerichtetes Magnetfeld kompensiert wird, so dass keine Anziehungskraft mehr auf die Partikel wirkt und diese von dem vorbeiströmenden Fluid mitgerissen werden. Um eine aufwändige Regelung zu vermeiden, die die Stromstärke in der Induktionsspule jeweils so abstimmt, dass das Magnetfeld des Permanentmagneten exakt kompensiert wird, ist erfindungsgemäß die Entladung des mindestens einen Kondensators über die Induktionsspule vorgesehen. Die Kapazität des mindestens einen Kondensators und die Induktivität der Spule sind dabei so aufeinander abgestimmt, dass bei der Entladung des vollständig aufgeladenen Kondensators zunächst ein maximales Magnetfeld induziert wird, welches stärker ist als das Magnetfeld des Permanentmagneten, sodass dieses "überkompensiert" wird. Da im weiteren Verlauf der Entladung die Spannung des mindestens einen Kondensators und damit die Stromstärke in der Induktionsspule kontinuierlich abnehmen, wird zwangsläufig zu einem bestimmten Zeitpunkt ein Bereich durchlaufen, in dem das Magnetfeld des Permanentmagneten genau kompensiert wird, d.h. die Summe der beiden Magnetfelder ist für eine bestimmte Zeitdauer so gering, dass die auf die ferromagnetischen Partikel wirkende Anziehungskraft geringer ist als die von dem vorbeiströmenden Fluid ausgeübte Kraft.

Die Reinigung der Sensorfläche durch die Entladung des mindestens einen Kondensators kann in Abhängigkeit von einem Signal des Detektors entweder manuell oder durch eine automatische Steuerung ausgelöst werden, wie dies weiter unten im Einzelnen beschrieben wird. In beiden Fällen kann die erfindungsgemäße Sensorvorrichtung kontinuierlich betrieben werden, ohne dass sie ausgetauscht oder zur Reinigung entfernt werden muss. Die Länge des Zeitraumes zwischen zwei erforderlichen Reinigungen stellt dabei ein Maß für die Partikelbelastung des Fluids dar.

Die Sensorfläche ist günstigerweise eine ebene Fläche, wobei die magnetischen Feldlinien des Permanentmagneten zumindest bereichsweise senkrecht zu der ebenen Fläche orientiert sind. Dadurch wirkt eine magnetische Anziehungskraft auf die ferromagnetischen Partikel in einem vorbeiströmenden Fluid, dem die Sensorfläche ausgesetzt ist, so dass sich die Partikel an der Sensorfläche anlagern. Die Strömungsrichtung des Fluids ist bevorzugt parallel zu der Sensorfläche.

Bevorzugt wird die Sensorfläche durch eine mit dem Permanentmagneten verbundene Magnetflussführung gebildet. Der Permanentmagnet steht also nicht in direktem Kontakt mit dem Fluid, sondern das Magnetfeld im Bereich der Sensorfläche wird mittelbar über die Magnetflussführung erzeugt. Die Magnetflussführung umfasst bevorzugt ein ferromagnetisches Material, insbesondere Eisen, und kann in ihrer Geometrie an die räumlichen Gegebenheiten am Einsatzort der Sensorvorrichtung angepasst werden.

Die Induktionsspule, die gemäß der Erfindung vorgesehen ist, kann den Permanentmagnet oder die gegebenenfalls vorgesehene Magnetflussführung umgeben. Der Permanentmagnet bzw. die Magnetflussführung bilden also zusammen mit der Induktionsspule einen Elektromagnet, dessen Magnetfeld dem Magnetfeld des Permanentmagneten entgegen gerichtet ist, wenn ein elektrischer Strom durch die Induktionsspule fließt.

Um die Entladung über die Induktionsspule zu ermöglichen, kann der mindestens eine Kondensator mit der Induktionsspule in Reihe oder parallel schaltbar sein, d.h. der mindestens eine Kondensator und die Induktionsspule können während des Entladungsvorgangs nach Art eines Resonanzkreises miteinander verschaltet sein.

Wie bereits angesprochen, muss die Kapazität des mindestens einen Kondensators auf die Induktivität der Spule abgestimmt sein, so dass zu Beginn des Entladevorgangs ein Magnetfeld induziert werden kann, welches das Magnetfeld des Permanentmagneten überkompensiert. Indirekt hängt die erforderliche Kapazität des mindestens einen Kondensators also auch von der Stärke des Permanentmagneten ab. Die Kapazität des mindestens einen Kondensators ist dabei relativ groß und liegt typischerweise im Bereich von mehreren Farad, je nach Spule z.B. im Bereich von ca. 3 F bis ca. 10 F. Bevorzugt umfasst der mindestens eine Kondensator einen Doppelschicht-Kondensator. Solche Kondensatoren werden typischerweise als Energiespeicher eingesetzt. Der Entladungsvorgang des mindestens einen Kondensators über die Induktionsspule dauert in der Regel wenige Sekunden, und ist u.a. abhängig von der Induktivität der Spule und gegebenenfalls einem zusätzlichen Dämpfungs- bzw. Windungswiderstand. Das Abklingen des induzierten Magnetfeldes während des Entladungsvorgangs von einem Maximalwert (bei dem das Magnetfeld des Permanentmagneten überkompensiert wird) bis auf null entspricht dabei einem Kriechfall oder einem aperiodischen Grenzfall.

Wenn die erfindungsgemäße Sensorvorrichtung mehr als einen Kondensator umfasst, können die Kondensatoren parallel geschaltet sein, um die Gesamtkapazität zu erhöhen, oder in Reihe, um die Spannung zu begrenzen, die an jedem einzelnen Kondensator während des Aufladevorgangs anliegt.

Bevorzugt umfasst die Sensorvorrichtung eine Stromquelle mit Spannungsbegrenzung, mittels derer der mindestens eine Kondensator aufladbar ist. Die Spannungsquelle umfasst insbesondere ein Netzteil, welches eine relativ niedrige Spannung (typischerweise ca. 2 bis 3 V) liefert, da geeignete Kondensatoren, insbesondere Doppelschicht-Kondensatoren, keine höheren Spannungen tolerieren. Des Weiteren ist es günstig, wenn die Stromquelle eine Begrenzung des Ladestroms ermöglicht.

Günstig ist es, wenn die Induktionsspule beim Aufladen des mindestens einen Kondensators stromlos schaltbar ist. Das Aufladen des Kondensators kann also unabhängig von der Induktionsspule erfolgen, sodass das Magnetfeld des Permanentmagneten durch den Aufladevorgang, der typischerweise einige Minuten in Anspruch nimmt, nicht beeinflusst wird. Dies gilt ebenso für die Zeit zwischen der Aufladung und der Entladung des mindestens einen Kondensators, während der sich zunehmend ferromagnetische Partikel an der Sensorfläche anlagern. Die Zeit zwischen zwei Reinigungen der Sensorfläche kann je nach Dimensionierung und Einsatzzweck der Sensorvorrichtung sowie der Partikelbelastung des Fluids einige Tage bis zu mehrere Wochen betragen. Während dieser Zeit kann der mindestens eine Kondensator bei Bedarf nachgeladen werden, falls es in Folge einer Selbstentladung zu einem Spannungsabfall kommt.

Der Detektor der erfindungsgemäßen Sensorvorrichtung, mit dem ferromagnetische Partikel im Bereich der Sensorfläche detektierbar ist, liefert ein Signal, das als Maß für die Belastung des Fluids mit ferromagnetischen Partikeln dient. Dies gilt allerdings nur, bis eine maximale Menge von Partikeln an der Sensorfläche angelagert ist, d.h. bis eine Art Sättigung eintritt. Detektoren, die im Rahmen der Erfindung eingesetzt werden können, umfassen z.B. Fe-Resonanzdetektoren, Interdigitalkondensatoren (IDK), Hall-Detektoren, Resistivdetektoren und optische Detektoren. Die verschiedenen Detektoren unterscheiden sich zum Teil in ihrer Empfindlichkeit und der maximal detektierbaren Partikelmenge, so dass ein geeigneter Detektor in Abhängigkeit von der zu erwartenden bzw. tolerierbaren Partikelbelastung der fluidtechnischen Anlage ausgewählt werden kann. Beispielsweise ist ein IDK relativ empfindlich und reagiert bereits auf die Anlagerung weniger Partikel, sodass er insbesondere in einer Sensorvorrichtung für Hydrauliksysteme eingesetzt werden kann. Demgegenüber kann ein weniger empfindlicher Hall-Detektor bei Systemen mit einem starken Abrieb eingesetzt werden, z.B. zur Detektion der Partikelbelastung von Getriebeöl.

Bei einer bevorzugten Ausführungsform der Erfindung umfasst die Sensorvorrichtung ferner ein mit dem Detektor verbundenes optisches oder akustisches Anzeigemittel, durch das ein Signal des Detektors anzeigbar ist. Das Anzeigemittel kann das Signal des Detektors entweder kontinuierlich anzeigen (z.B. in Form einer Digitalanzeige oder eines Zeigerinstruments), oder das Anzeigemittel kann lediglich anzeigen, wenn das Signal einen vorgegebenen Schwellenwert erreicht (z.B. in Form einer Kontrollleuchte oder eines Signaltons). Der vorgegebene Schwellenwert entspricht einer bestimmten Menge an Partikeln, die an der Sensorfläche angelagert sind, wobei diese Partikelmenge kleiner oder gleich der maximalen Kapazität der Sensorfläche (Sättigung) sein kann. Das Anzeigemittel kann ein kontinuierliches Signal des Detektors (z.B. eine Spannung oder eine Stromstärke) direkt anzeigen oder indirekt über einen Signalwandler, eine Schnittstelle oder dergleichen.

Bevorzugt umfasst die Sensorvorrichtung eine Schalteinrichtung, mittels derer die Entladung des mindestens einen Kondensators über die Induktionsspule manuell einleitbar ist. Bei dieser Ausführungsform kann der Benutzer bei Bedarf eine Reinigung der Sensorfläche durchführen, indem er z.B. einen Schalter oder Taster betätigt, wodurch der mindestens eine Kondensator entladen und anschließend wieder aufgeladen wird. Die Entscheidung, wann eine Reinigung erforderlich ist, kann der Benutzer anhand des optischen oder akustischen Anzeigemittels treffen, wobei er bei einer kontinuierlichen Anzeige des Detektorsignals die Möglichkeit hat, einen geeigneten Schwellenwert jeweils selbst festzulegen.

Gemäß einer weiteren bevorzugten Ausführungsform der Erfindung umfasst die Sensorvorrichtung eine mit dem Detektor verbundene Steuerungseinheit, mittels derer die Entladung des mindestens einen Kondensators in Abhängigkeit von einem Signal des Detektors steuerbar ist. In diesem Fall erfolgt die Reinigung der Sensorfläche automatisch, indem durch die Steuerungseinheit jeweils eine Entladung des Kondensators eingeleitet wird, sobald das Detektorsignal einen vorgegebenen Schwellenwert erreicht. Anzeigemittel für das Detektorsignal sind hierbei nicht unbedingt erforderlich, wobei optional vorgesehen sein kann, dass die Reinigung der Sensorfläche jeweils angezeigt wird. Besonders günstig ist es, wenn die Zeitdauer zwischen zwei aufeinander folgenden Reinigungen gespeichert und gegebenenfalls angezeigt wird, da diese Zeiten ein Maß für die Partikelbelastung des Fluids darstellen. Eine zunehmende Verkürzung der Reinigungszyklen deutet darauf hin, dass die Verschmutzung des Fluids mit ferromagnetischen Partikeln zunimmt und gegebenenfalls entsprechende Maßnahmen (z.B. Austausch von Filtern) eingeleitet werden müssen.

Das Fluid im Rahmen der vorliegenden Erfindung ist insbesondere ein Schmiermittel oder eine Hydraulikflüssigkeit, d.h. die erfindungsgemäße Sensorvorrichtung kann eingesetzt werden zur Detektion von ferromagnetischen Partikeln im Schmiermittelkreislauf von Getrieben oder Motoren (sowohl in Fahrzeugen als auch stationären Anlagen), oder in diversen hydraulischen Systemen. Die Sensorvorrichtung kann jeweils im Hauptkreislauf oder in einem Nebenkreislauf des entsprechenden Systems angeordnet sein.

Gegenstand der vorliegenden Erfindung ist ferner ein Verfahren zum Betrieb der vorstehend beschriebenen Sensorvorrichtung, wobei die folgenden Schritte wiederholt durchgeführt werden:
- Aufladen des mindestens einen Kondensators;
- Entladen des mindestens einen Kondensators über die Induktionsspule, sobald ein Signal des Detektors einen vorgegebenen Schwellenwert erreicht; und
- Erfassen der Zeiträume zwischen den Entladungen.

Besondere Vorteile sowie bevorzugte Ausführungsformen des erfindungsgemäßen Verfahrens wurden bereits im Zusammenhang mit der erfindungsgemäßen Sensorvorrichtung erläutert. Das Verfahren kann insbesondere automatisch durchgeführt werden, indem das Auf- und Entladen des mindestens einen Kondensators mittels einer Steuerungseinheit in Abhängigkeit vom Signal des Detektors gesteuert werden.

Bevorzugt wird die Induktionsspule während der Aufladung des mindestens einen Kondensators stromlos geschaltet. Dadurch wird eine Beeinflussung des Magnetfeldes des Permanentmagneten während der Aufladung des mindestens einen Kondensators vermieden.

Diese und weitere Vorteile der Erfindung werden anhand der nachfolgenden Ausführungsbeispiele unter Bezugnahme auf die Figuren näher erläutert. Es zeigen im Einzelnen:
- Figur 1:: den prinzipiellen Aufbau einer erfindungsgemäßen Sensorvorrichtung;
- Figur 2:: ein Schaltbild eines ersten Ausführungsbeispiels einer erfindungsgemäßen Sensorvorrichtung; und
- Figur 3:: ein Schaltbild eines zweiten Ausführungsbeispiels einer erfindungsgemäßen Sensorvorrichtung.

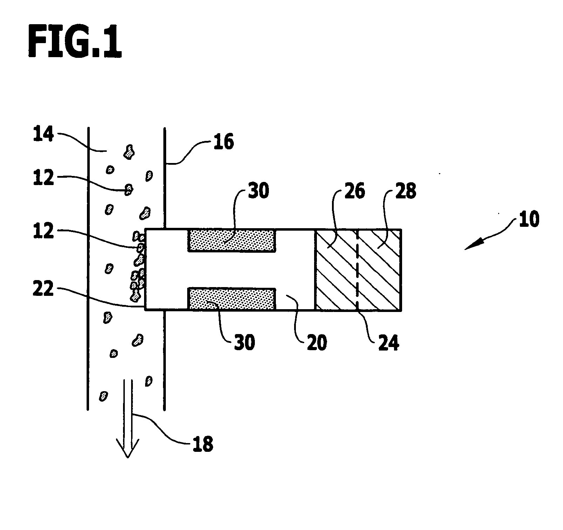

Figur 1 zeigt schematisch den prinzipiellen Aufbau einer Sensorvorrichtung 10 gemäß der vorliegenden Erfindung zur Detektion von ferromagnetischen Partikeln 12 in einem Fluid 14. Das Fluid 14 (z.B. ein Schmiermittel oder eine Hydraulikflüssigkeit) befindet sich in einem Fluidkreislauf und bewegt sich in einer Leitung 16 entlang einer Strömungsrichtung 18.

Die Sensorvorrichtung 10 umfasst eine Magnetflussführung 20, die seitlich in die Leitung 16 hineinragt. Eine Stirnfläche der Magnetflussführung 20 bildet eine ebene Sensorfläche 22, die dem Fluid 14 ausgesetzt ist und parallel zu der Strömungsrichtung 18 orientiert ist. Die entgegengesetzte Stirnfläche der Magnetflussführung 20 ist mit einem Permanentmagnet 24 mit einem Nordpol 26 und einem Südpol 28 verbunden. Der Permanentmagnet 24 erzeugt über die Magnetflussführung 20 ein Magnetfeld im Bereich der Sensorfläche 22, dessen Feldlinien zumindest bereichsweise senkrecht zu der ebenen Sensorfläche 22 orientiert sind. Dadurch werden ferromagnetische Partikel 12 angezogen und lagern sich an der Sensorfläche 22 an.

Die Sensorvorrichtung 10 umfasst ferner eine Induktionsspule 30, die die Magnetflussführung 20 umgibt. Mit der Induktionsspule 30 ist ein Magnetfeld induzierbar, das dem Magnetfeld des Permanentmagneten 24 entgegen gerichtet ist.

Die Sensorvorrichtung 10 umfasst noch eine Reihe weiterer Komponenten, die in der Figur 1 aus Gründen der Übersichtlichkeit nicht dargestellt sind, und deren Funktion sich aus den nachfolgend beschriebenen Schaltbildern ergibt.

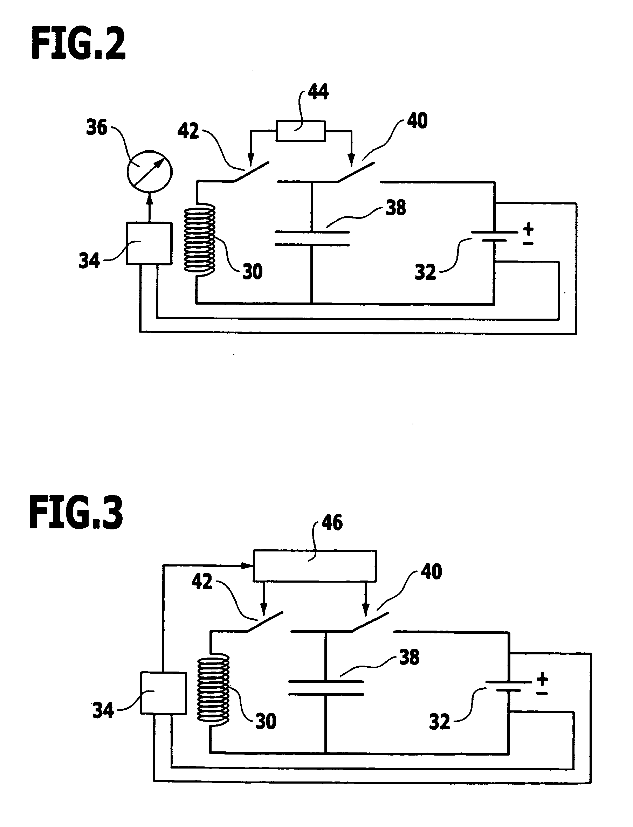

Die Figur 2 zeigt ein schematisches Schaltbild eines ersten Ausführungsbeispiels der erfindungsgemäßen Sensorvorrichtung 10.

Die Sensorvorrichtung 10 umfasst eine Stromquelle 32 mit Spannungsbegrenzung (z.B. ein Netzteil), an die ein Detektor 34 angeschlossen ist. Mit dem Detektor 34 sind die im Bereich der Sensorfläche 22 angelagerten ferromagnetischen Partikel 12 detektierbar. Bei dem Detektor 34 kann es sich z.B. um einen Fe-Resonanzdetektor, einen IDK, einen Hall-Detektor, einen Resistivdetektor oder einen optischen Detektor handeln.

Mit dem Detektor 34 ist ein optisches oder akustisches Anzeigemittel 36 verbunden zur Anzeige eines Signals des Detektors 34, welches ein Maß für die Menge der an der Sensorfläche 22 angelagerten Partikel 12 darstellt. Das Anzeigemittel 36 kann das Detektorsignal kontinuierlich anzeigen (z.B. als Zeigerinstrument) oder nur dann, wenn ein vorgegebener Schwellenschwert überschritten wird (z.B. als LED).

Die Sensorvorrichtung 10 umfasst ferner einen Kondensator 38, der durch die Stromquelle 32 aufgeladen werden kann, wenn ein erster Schalter 40 geschlossen ist. Der Kondensator 38 wird über die Induktionsspule 30 entladen, wenn ein zweiter Schalter 42 geschlossen ist.

Der Kondensator 38 weist eine ausreichend hohe Kapazität auf, je nach Art der Induktionsspule 30 z.B. im Bereich von ca. 3 bis ca. 10 F . Geeignet ist hierfür z.B. ein Doppelschicht-Kondensator. Wenn der zweite Schalter 42 geschlossen und der Kondensator 38 über die Induktionsspule 30 entladen wird, wird durch die Spule 30 zu Beginn ein maximales Magnetfeld induziert, welches entgegen gerichtet und stärker ist als das Magnetfeld des Permanentmagneten 24, d.h. dieses wird zunächst überkompensiert. Die Spannung des Kondensators 38 sowie die Stromstärke in der Spule 30 fallen im weiteren Verlauf der Entladung kontinuierlich ab, sodass das induzierte Magnetfeld zwangsläufig einen Bereich durchläuft, in dem das Magnetfeld des Permanentmagneten 24 genau kompensiert wird. Dabei wird die auf die Partikel 12 wirkende Anziehungskraft aufgehoben und die Sensorfläche 22 von den Partikeln 12 gereinigt. Der gesamte Entladungsvorgang des Kondensators 38 dauert in der Regel nur wenige Sekunden.

Während der Aufladung des Kondensators 38 durch die Stromquelle 32 ist der zweite Schalter 42 geöffnet, sodass die Induktionsspule 30 während der Aufladung stromlos ist.

Während des Betriebs der Sensorvorrichtung 10 zeigt das Anzeigemittel 36 kontinuierlich das Signal des Detektors 34 an und/oder signalisiert das Erreichen des Schwellenwertes. Sobald dies der Fall ist, kann der Benutzer durch Betätigen einer Schalteinrichtung 44 kurzzeitig den ersten Schalter 40 öffnen und den zweiten Schalter 42 schließen, so dass durch die Entladung des Kondensators 38 über die Induktionsspule 30 die Sensorfläche 22 gereinigt wird. Anschließend wird wieder der erste Schalter 40 geschlossen und der zweite Schalter 42 geöffnet, der Kondensator 38 wird wieder aufgeladen und es beginnen sich wieder Partikel 12 an der Sensorfläche 22 anzulagern. Die Länge der Zeiträume zwischen zwei erforderlichen Betätigungen der Schalteinrichtung 44 sind ein Maß für die Partikelbelastung des Fluids 14.

Die Figur 3 zeigt ein schematisches Schaltbild eines zweiten Ausführungsbeispiels der Sensorvorrichtung 10.

Im Unterschied zum ersten Ausführungsbeispiel gemäß der Figur 2 ist in diesem Fall eine mit dem Detektor 34 verbundene Steuerungseinheit 46 vorgesehen, mittels derer die Entladung des Kondensators 38 in Abhängigkeit von einem Signal des Detektors 34 steuerbar ist. Die Steuerungseinheit 46 ersetzt somit die manuelle Betätigung einer Schalteinrichtung, so dass die Sensorvorrichtung 10 über einen längeren Zeitraum automatisch betrieben werden kann. Sobald das Signal des Detektors 34 einen vorgegebenen Schwellenwert erreicht, wird durch die Steuerungseinheit 46 für kurze Zeit der erste Schalter 40 geöffnet und der zweite Schalter 42 geschlossen. Die Entladung des Kondensators 38 über die Induktionsspule 30 erfolgt dann wie oben beschrieben.

Die Länge der Zeiträume zwischen zwei Entladungen des Kondensators 38 werden von der Steuerungseinheit 46 erfasst und gespeichert und sind ein Maß für die Partikelbelastung des Fluids 14. Wenn diese Zeiträume kürzer werden, ist die Verschmutzung des Fluids 14 angestiegen. Ein Anzeigemittel für das Signal des Detektors 34 ist in diesem Fall nicht erforderlich, kann jedoch optional vorgesehen sein.

### Bezugszeichenliste

- 10: Sensorvorrichtung
- 12: ferromagnetische Partikel
- 14: Fluid
- 16: Leitung
- 18: Strömungsrichtung
- 20: Magnetflussführung
- 22: Sensorfläche
- 24: Permanentmagnet
- 26: Nordpol
- 28: Südpol
- 30: Induktionsspule
- 32: Stromquelle
- 34: Detektor
- 36: Anzeigemittel
- 38: Kondensator
- 40: erster Schalter
- 42: zweiter Schalter
- 44: Schalteinrichtung
- 46: Steuerungseinheit

## Patentansprüche

1. Sensorvorrichtung (10) zur Detektion von ferromagnetischen Partikeln (12) in einem Fluid (14), umfassend
- eine Sensorfläche (22), die dem Fluid (14) ausgesetzt ist;
- einen Permanentmagnet (24), der ein Magnetfeld im Bereich der Sensorfläche (22) erzeugt;
- einen Detektor (34), mit dem ferromagnetische Partikel im Bereich der Sensorfläche (22) detektierbar sind;
- eine Induktionsspule (30), mit der ein Magnetfeld induzierbar ist, das dem Magnetfeld des Permanentmagneten (24) entgegen gerichtet ist; und
- mindestens einen Kondensator (38), der so eingerichtet ist, dass er über die Induktionsspule (30) entladbar ist, wobei durch die Entladung des mindestens einen Kondensators (38) über die Induktionsspule (30) ein maximales Magnetfeld induzierbar ist, welches stärker ist als das Magnetfeld des Permanentmagneten (24).

2. Sensorvorrichtung (10) nach Anspruch 1, wobei die Sensorfläche (22) eine ebene Fläche ist, und wobei die magnetischen Feldlinien des Permanentmagneten (24) zumindest bereichsweise senkrecht zu der ebenen Fläche orientiert sind.

3. Sensorvorrichtung (10) nach Anspruch 1 oder 2, wobei die Sensorfläche (22) durch eine mit dem Permanentmagnet (24) verbundene Magnetflussführung (20) gebildet wird.

4. Sensorvorrichtung (10) nach einem der vorangehenden Ansprüche, wobei die Induktionsspule (30) den Permanentmagnet (24) oder die Magnetflussführung (20) umgibt.

5. Sensorvorrichtung (10) nach einem der vorangehenden Ansprüche, wobei der mindestens eine Kondensator (38) mit der Induktionsspule (30) in Reihe oder parallel schaltbar ist.

6. Sensorvorrichtung (10) nach einem der vorangehenden Ansprüche, wobei der mindestens eine Kondensator (38) einen Doppelschicht-Kondensator umfasst.

7. Sensorvorrichtung (10) nach einem der vorangehenden Ansprüche, ferner umfassend eine Stromquelle (32) mit Spannungsbegrenzung, mittels derer der mindestens eine Kondensator (38) aufladbar ist.

8. Sensorvorrichtung (10) nach Anspruch 7, wobei die Induktionsspule (30) beim Aufladen des mindestens einen Kondensators (38) stromlos schaltbar ist.

9. Sensorvorrichtung (10) nach einem der vorangehenden Ansprüche, wobei der Detektor (34) einen Fe-Resonanzdetektor, einen Interdigitalkondensator (IDK), einen Hall-Detektor, einen Resistivdetektor oder einen optischen Detektor umfasst.

10. Sensorvorrichtung (10) nach einem der vorangehenden Ansprüche, ferner umfassend ein mit dem Detektor (34) verbundenes optisches oder akustisches Anzeigemittel (36), durch das ein Signal des Detektors (34) anzeigbar ist.

11. Sensorvorrichtung (10) nach einem der vorangehenden Ansprüche, ferner umfassend eine Schalteinrichtung (44), mittels derer die Entladung des mindestens einen Kondensators (38) über die Induktionsspule (30) manuell einleitbar ist.

12. Sensorvorrichtung (10) nach einem der vorangehenden Ansprüche, ferner umfassend eine mit dem Detektor (34) verbundene Steuerungseinheit (46), mittels derer die Entladung des mindestens einen Kondensators (38) in Abhängigkeit von einem Signal des Detektors (34) steuerbar ist.

13. Sensorvorrichtung (10) nach einem der vorangehenden Ansprüche, wobei das Fluid (14) ein Schmiermittel, insbesondere ein Getriebe- oder Motoröl, oder eine Hydraulikflüssigkeit ist.

14. Verfahren zum Betrieb einer Sensorvorrichtung (10) nach einem der vorangehenden Ansprüche, wobei die folgenden Schritte wiederholt durchgeführt werden:
- Aufladen des mindestens einen Kondensators (38);
- Entladen des mindestens einen Kondensators (38) über die Induktionsspule (30), sobald ein Signal des Detektors (34) einen vorgegebenen Schwellenwert erreicht; und
- Erfassen der Zeiträume zwischen den Entladungen.

15. Verfahren nach Anspruch 14, wobei die Induktionsspule (30) während der Aufladung des mindestens einen Kondensators (38) stromlos geschaltet wird.

## Claims

1. A sensor device (10) for detecting ferromagnetic particles (12) in a fluid (14), comprising
- a sensor surface (22), which is exposed to the fluid (14);
- a permanent magnet (24), which generates a magnetic field in the region of the sensor surface (22);
- a detector (34), with which ferromagnetic particles in the region of the sensor surface (22) are detectable;
- an induction coil (30), with which a magnetic field is inducible that is oriented opposite to the magnetic field of the permanent magnet (24); and
- at least one capacitor (38), which is configured to be dischargeable via the induction coil (30), wherein a maximum magnetic field is inducible upon discharging of the at least one capacitor (38) via the induction coil (30) that is stronger than the magnetic field of the permanent magnet (24).

2. The sensor device (10) according to claim 1, wherein the sensor surface (22) is a flat surface, and wherein the magnetic flux lines of the permanent magnet (24) are oriented, at least in one area, perpendicularly to the flat surface.

3. The sensor device (10) according to claim 1 or 2, wherein the sensor surface (22) is formed by a magnetic flux conduct (20) connected to the permanent magnet (24).

4. The sensor device (10) according to one of the preceding claims,
wherein the induction coil (30) surrounds the permanent magnet (24) or the magnetic flux conduct (20).

5. The sensor device (10) according to one of the preceding claims,
wherein the at least one capacitor (38) can be connected to the induction coil (30) in series or in parallel.

6. The sensor device (10) according to one of the preceding claims,
wherein the at least one capacitor (38) comprises a double-layer capacitor.

7. The sensor device (10) according to one of the preceding claims, further comprising a current source (32) with voltage limitation, by means of which the at least at one capacitor (38) can be charged.

8. The sensor device (10) according to claim 7, wherein the induction coil (30) can be switched currentless when charging the at least one capacitor (38).

9. The sensor device (10) according to one of the preceding claims,
wherein the detector (34) comprises an Fe-resonance detector, an interdigital capacitor (IDC), a Hall-detector, a resistive detector or an optical detector.

10. The sensor device (10) according to one of the preceding claims, further comprising an optical or acoustical display means (36) connected to the detector (34), by means of which a signal from the detector (34) can be displayed.

11. The sensor device (10) according to one of the preceding claims, further comprising a switching device (44), by means of which the discharging of the at least one capacitor (38) via the induction coil (30) can be initiated manually.

12. The sensor device (10) according to one of the preceding claims, further comprising a control unit (46) connected to the detector (34), by means of which the discharging of the at least one capacitor (38) can be controlled as a function of a signal from the detector (34).

13. The sensor device (10) according to one of the preceding claims,
wherein the fluid (14) is a lubricant, in particular a motor oil or gear oil, or a hydraulic fluid.

14. A method for operating a sensor device (10) according to one of the preceding claims, wherein the following steps are performed repeatedly:
- charging the at least one capacitor (38);
- discharging the at least one capacitor (38) via the induction coil (30) as soon as a signal from the detector (34) has reached a given threshold; and
- detecting the time intervals between the discharges.

15. The method according to claim 14, wherein the induction coil (30) is switched currentless during the charging of the at least one capacitor (38).

## Revendications

1. Dispositif de capteur (10) destiné à la détection de particules ferromagnétiques (12) dans un fluide (14), comprenant
- une surface de détection (22) qui est exposée au fluide (14) ;
- un aimant permanent (24) qui produit un champ magnétique dans la zone de la surface de détection (22) ;
- un détecteur (34) avec lequel des particules ferromagnétiques peuvent être détectées dans la zone de la surface de détection (22) ;
- une bobine d'induction (30) avec laquelle il est possible d'induire un champ magnétique qui est orienté à l'opposé du champ magnétique de l'aimant permanent (24) ; et
- au moins un condensateur (38) qui est conçu de sorte qu'il puisse être déchargé par le biais de la bobine d'induction (30), dans lequel la décharge dudit au moins un condensateur (38) par le biais de la bobine d'induction (30) peut induire un champ magnétique maximal qui est plus fort que le champ magnétique de l'aimant permanent (24).

2. Dispositif de capteur (10) selon la revendication 1, dans lequel la surface de détection (22) est une surface plane et dans lequel les lignes de champ magnétiques de l'aimant permanent (24) sont orientées au moins par zones perpendiculairement à la surface plane.

3. Dispositif de capteur (10) selon la revendication 1 ou 2, dans lequel la surface de détection (22) est formée par une commande du flux magnétique (20) liée à l'aimant permanent (24).

4. Dispositif de capteur (10) selon l'une quelconque des revendications précédentes, dans lequel la bobine d'induction (30) entoure l'aimant permanent (24) ou la commande du flux magnétique (20).

5. Dispositif de capteur (10) selon l'une quelconque des revendications précédentes, dans lequel ledit au moins un condensateur (38) peut être monté en série ou en parallèle avec la bobine d'induction (30).

6. Dispositif de capteur (10) selon l'une quelconque des revendications précédentes, dans lequel ledit au moins condensateur (38) comprend un condensateur à double couche.

7. Dispositif de capteur (10) selon l'une quelconque des revendications précédentes, comprenant en outre une source de courant (32) avec limitation de tension au moyen de laquelle ledit au moins un condensateur (38) peut être chargé.

8. Dispositif de capteur (10) selon la revendication 7, dans lequel le courant de la bobine d'induction (30) peut être coupé lors de la charge dudit au moins un condensateur (38).

9. Dispositif de capteur (10) selon l'une quelconque des revendications précédentes, dans lequel le détecteur (34) comprend un détecteur à ferrorésonance, un condensateur interdigité (IDK), un détecteur à effet Hall, un détecteur résistif ou un détecteur optique.

10. Dispositif de capteur (10) selon l'une quelconque des revendications précédentes, comprenant en outre un moyen d'indication (36) optique ou acoustique, relié au détecteur (34), par lequel un signal du détecteur (34) peut être indiqué.

11. Dispositif de capteur (10) selon l'une quelconque des revendications précédentes, comprenant en outre un dispositif de commutation (44) au moyen duquel la décharge dudit au moins un condensateur (38) peut être déclenchée manuellement par le biais de la bobine d'induction (30).

12. Dispositif de capteur (10) selon l'une quelconque des revendications précédentes, comprenant en outre une unité de commande (46) reliée au détecteur (34) au moyen de laquelle la décharge dudit au moins un condensateur (38) peut être commandée en fonction d'un signal du détecteur (34).

13. Dispositif de capteur (10) selon l'une quelconque des revendications précédentes, dans lequel le fluide (14) est une matière lubrifiante, en particulier une huile à engrenages ou une huile pour moteurs, ou un liquide hydraulique.

14. Procédé de fonctionnement d'un dispositif de capteur (10) selon l'une quelconque des revendications précédentes, dans lequel les étapes suivantes sont exécutées de façon répétée :
- charge dudit au moins un condensateur (38) ;
- décharge dudit au moins condensateur (38) par le biais de la bobine d'induction (30) dès qu'un signal du détecteur (34) atteint une valeur de seuil prédéfinie ; et
- détection des intervalles de temps entre les décharges.

15. Procédé selon la revendication 14, dans lequel le courant de la bobine d'induction (30) est coupé durant la charge dudit au moins condensateur (38).
